# EUROPEAN PATENT APPLICATION

(11) **EP 4 209 599 A1**
(43) Date of publication of application: **12.07.2023**
(21) Application number: 21864684.2
(22) Date of filing: 02.09.2021
(51) Int. Cl.: C12Q 1/6886, A61K 38/19, A61P 35/00, A23L 33/135

(54) **METHOD FOR SCREENING IMMUNOGENIC ANTICANCER ACTIVITY CYTOKINE, AND COMPOSITION FOR PREVENTING OR TREATING CANCER DISEASE, COMPRISING IL-15 AS ACTIVE INGREDIENT**

(30) Priority: 04.09.2020 KR 20200112774; 30.08.2021 KR 20210114651
(71) Applicant: Daegu Gyeongbuk Institute Of Science and Technology, Daegu 42988 (KR); Kyungpook National University Industry-Academic Cooperation Foundation, Daegu 41566 (KR)
(72) Inventor: BAEK, Moon-Chang, Daegu 42116 (KR); YEA, Kyung Moo, Daegu 43016 (KR); KANG, Sung-Min, Daegu 42804 (KR)
(74) Representative: Roos, Peter
(86) International application number: PCT/KR2021/011864
(87) International publication number: WO 2022/050725

(57) **Abstract**

The present invention relates to a method for screening a cytokine having anticancer activity that inhibits the exosome secretion or suppresses the expression of PD-L1, wherein a vector composed of gene encoding cytokine, linker domain, and transmembrane domain transforms into cancer cells, and cytokine exhibiting anticancer activity can be selected, and thus the selected cytokine is provided as a new anticancer agent. In addition, the present invention relates to composition for preventing or treating cancer diseases, the composition comprising IL-15, selected using the above-mentioned screening technique, or expression promoter or activator thereof as an active ingredient. In the present invention, it was found that treating cancer cells with IL-15 suppresses exosome secretion and the expression of PD-L1, and it was also found that the anticancer effect is directly exhibited on the cancer cells. Therefore, IL-15 or expression promoter or activator can be effectively used to prevent or treat cancer diseases.

## Description

### Technical Field

The present disclosure relates to a method for screening immunogenic anticancer activity cytokines, and to a method for screening cytokines having immunogenic anticancer activity to inhibit exosome secretion of cancer cells or suppress expression of programmed apoptosis-ligand 1.

The present disclosure relates to a composition for preventing or treating cancer diseases, including IL-15 selected by screening, or an expression promoter or activator thereof as an active ingredient.

### Background Art

Cancer is a disease caused by abnormal cell growth, with reports that the mortality rate of cancer patients is high that it ranks first as the cause of death in Korea. There are dozens of cancer types, mainly classified on the basis of the tissue of origin. Cancer is divided into benign tumors and malignant tumors, wherein benign tumors have relatively slow growth rate and hardly show metastasis from the primary site of the tumor to other tissues, while malignant tumors are life-threatening due to rapid growth by infiltrating into other tissues away from the primary site.

Surgery, chemotherapy, and radiotherapy are applied for the cancer treatment methods, but it has been reported that more than 50% of cancer patients who had undergone treatment die despite prolonged research. The reason why it is difficult to treat cancer despite of application of various treatment methods is that even if the cancer is surgically removed to cure the cancer patient, cancer recurs due to the metastasis of the cancer cells to other tissues of the patient, or the prognosis of cancer deteriorates drastically due to cancer cells showing resistance to anticancer drugs during or after the treatment, although the size of the tumor decreases due to anticancer drugs in the initial treatment stage.

In particular, anticancer drugs, which are commonly used chemotherapy, show anticancer effects mainly through the mechanism of inhibiting the proliferation of cancer cells, and about 60 different types have been developed. However, most anticancer drugs are unable to selectively eliminate cancer cells only, causing side effects that affect normal cells. In addition, these chemotherapy anticancer drugs are not effective in cancer treatment due to resistance developed in cancer cells.

In order to overcome the issues of conventional anticancer drugs, targeted anticancer drugs and anticancer therapy using immune cells have been developed as treatment methods that specifically act on cancer cells. However, these treatment methods show discrepancies in treatment efficiency depending on the characteristics of the patient, such that the problem still remains unsolved, such as the development of acute inflammatory diseases due to excessive activation of the immune system. Therefore, in order to prevent the occurrence of cancer as well as treatment of cancer, it is urgent to develop new treatment methods that are less toxic but specific to cancer cells without inducing resistance in cancer cells.

On the other hand, exosomes are membranous small vesicles, secreted by most cells in the body. The diameter of exosomes is approximately 30-200 nm, and various kinds of genetic materials (DNA, RNA, miRNA), proteins, and lipids derived from the cell are included in the exosome. Exosomes originate from specific compartments in the cell, called multivesicular bodies (MVBs), and are released and secreted outside the cell, rather than falling off directly from the plasma membrane. In particular, exosomes secreted by cancer cells are known to affect cancer metastasis, neovascularization, and proliferation of cancer cells in the progression of cancer. Inhibition of exosome secretion in these cancer cells may degrade the function of exosomes in the progression of cancer, thereby inhibiting proliferation and metastasis of cancer cells.

In addition, programmed cell death 1 (PD-1), one of the immune checkpoint molecules, has been a therapeutic target in several cancers. PD-1 is upregulated in T cells upon activation and is abundant in exhausted T cells that are commonly seen in tumor-infiltrating lymphocytes. If the interaction between PD-1 and its ligand, programmed cell death-ligand 1 (PD-L1), is blocked, superior antitumor response and clinical effects were able be seen in some patients. Recently, research and development for various immunotherapy-based cancer treatment methods have been undertaken. In addition, it has been revealed that immunotherapies approved by the FDA derived positive outcomes in the treatment of various cancers. However, there is a shortcoming that its applicable range is limited to certain patients because it requires high cost for treatment, and consistent responsiveness is not induced in all patients. Therefore, it is necessary to research and develop a new concept of anticancer drug that may maximize the proliferation, metastasis, and therapeutic efficacy of cancer by using cytokines that exhibit immune checkpoint inhibitory effects and exosome secretion inhibitory effects.

### [Prior Art Document]

### [Patent Document]

Korean Patent Publication No. 10-2018-0090122

### Disclosure of the Invention

### Technical Goals

An object of the present disclosure is to provide a method for screening cytokines having immunogenic anticancer activity, by transforming cancer cells with lentivirus including a vector which includes a gene encoding a cytokine, a linker domain encoding a linker, and a transmembrane domain encoding a transmembrane protein.

Another object of the present disclosure is to provide a pharmaceutical composition for preventing or treating cancer diseases.

Another object of the present disclosure is to provide a method for inhibiting secretion of cancer cell-derived exosomes and PD-L1 expression.

Another object of the present disclosure is to provide a method for increasing secretion of immune cell-derived exosomes and suppressing expression of PD-1 and CTLA-4. Technical Solutions

The present disclosure provides a method for screening immunogenic anticancer activity cytokines, including (1) preparing a vector including a gene encoding a cytokine, a linker domain encoding a linker, and a transmembrane domain encoding a transmembrane protein; (2) preparing lentivirus including the vector prepared in operation (1); (3) transforming cancer cells with lentivirus prepared in operation (2); and, (4) when an expression level of a gene associated with exosome secretion or an expression level of PD-L1 in the cancer cells transformed in operation (3) decreases compared to untransformed control cells, determining that the cytokine in operation (1) has immunogenic anticancer activity.

In addition, the present disclosure provides a pharmaceutical composition for preventing or treating cancer, including an immunogenic anticancer activity cytokine selected by the screening method as an active ingredient.

In addition, the present disclosure provides a pharmaceutical composition for preventing or treating cancer diseases, including IL-15, or an expression promoter or activator thereof as an active ingredient.

In addition, the present disclosure provides a method for inhibiting secretion of cancer cell-derived exosomes and PD-L1 expression, including administering IL-15, or an expression promoter or activator thereof to a subject.

In addition, the present disclosure provides a method for increasing secretion of immune cell-derived exosomes and inhibiting expression of PD-1 and CTLA-4, including administering IL-15, or an expression promoter or activator thereof to a subject. Advantageous Effects

According to the present disclosure, by transforming cancer cells with a vector including a gene encoding a cytokine, a linker domain encoding a linker, and a transmembrane domain encoding a transmembrane protein, it is possible to select cytokines that exhibit immunogenic anticancer activity to inhibit the expression level of a gene associated with exosome secretion or the expression level of PD-L1, and the selected cytokines may be provided as a new anticancer drug.

In addition, in the present disclosure, it was found that, when IL-15 selected by screening was treated to cancer cells, the anticancer effect was enhanced as secretion of cancer cell-derived exosomes is inhibited while expression of PD-L1, which induces immune evasion of cytotoxic T cells, is suppressed, and it was also determined that IL-15 exhibits the anticancer effect directly on cancer cells. At the same time, it was found that the immunogenic anticancer effect was derived as IL-15 activates cytotoxic T cells, suppresses expression of PD-1 and CTLA-4, which are immune checkpoints, to reduce immune evasion of cancer cells, and enhances exosome secretion of cytotoxic T cells, such that IL-15, or an expression promoter or activator thereof may be useful as a composition for preventing or treating cancer diseases.

### Brief Description of Drawings

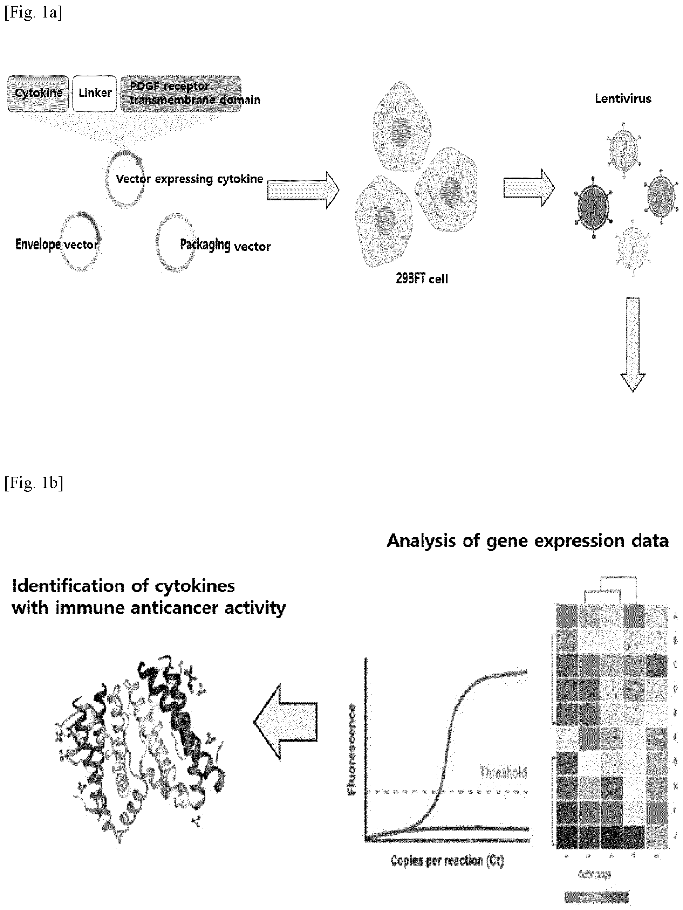

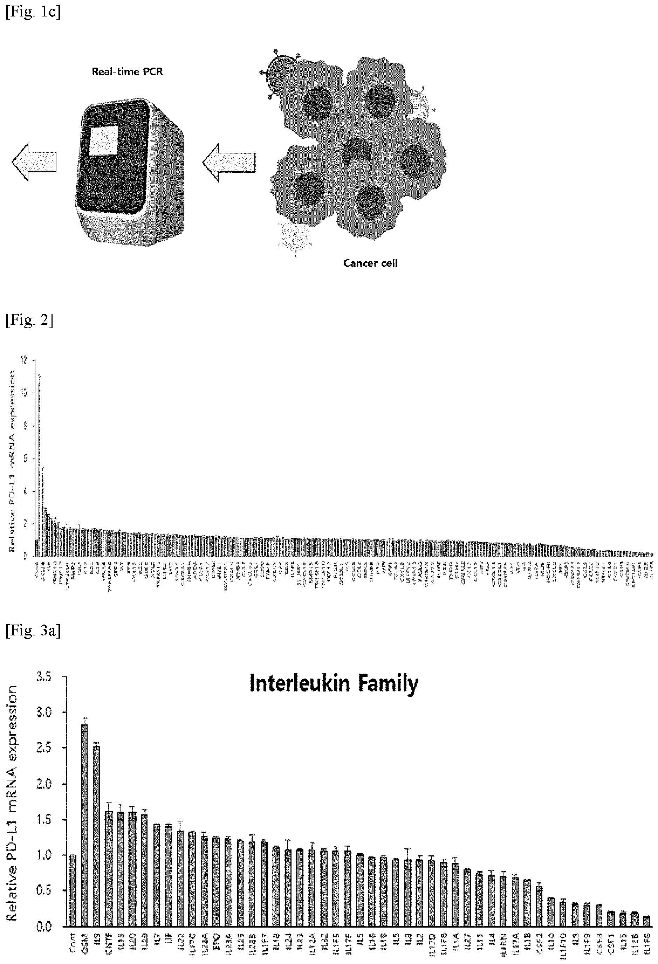

FIG. 1 shows a method for screening immunogenic anticancer activity cytokines by transforming cancer cells with a vector consisting of a gene encoding a cytokine, a linker domain encoding a linker, and a transmembrane domain encoding a transmembrane protein.
FIG. 2 shows a result of identifying changes in the expression level of PD-L1 in cancer cells with 202 kinds of cytokines expressed on the surface according to a method for screening immunogenic anticancer activity cytokines of the present disclosure.

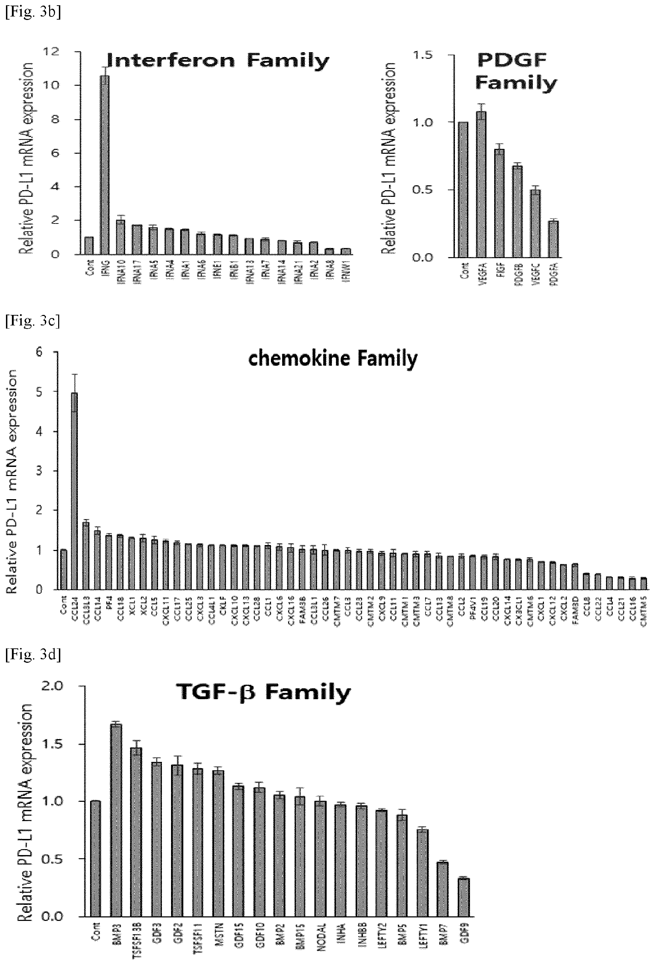

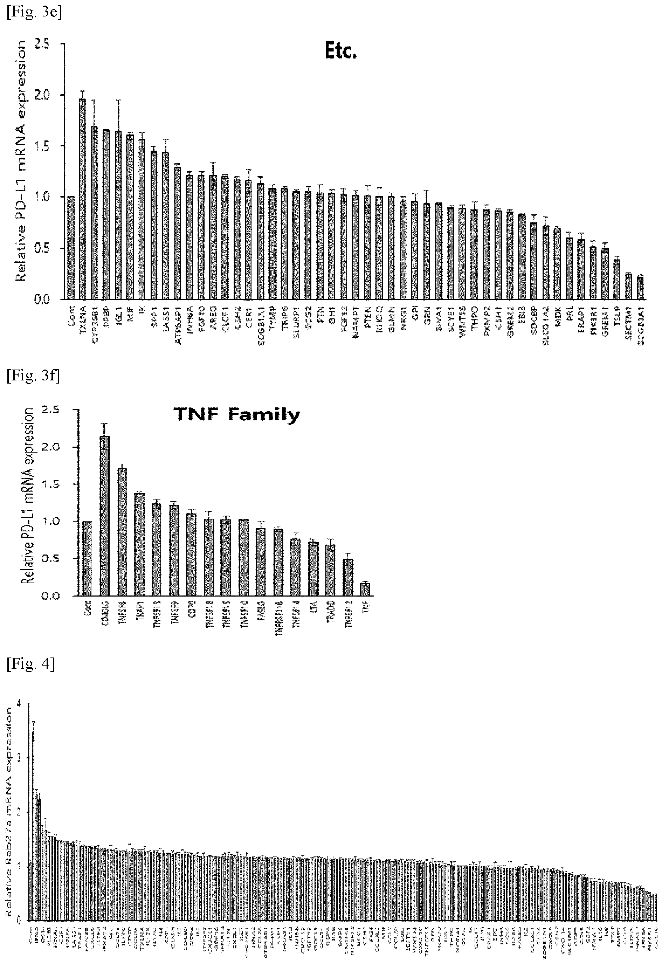

FIG. 3 shows results of classifying cancer cells with 202 kinds of cytokines expressed on the surface by family of cytokines according to a method for screening immunogenic anticancer activity cytokines of the present disclosure and identifying changes in the expression level of PD-L1.
FIG. 4 shows a result of identifying changes in the expression level of a gene associated with exosome secretion in cancer cells with 202 kinds of cytokines expressed on the surface according to a method for screening immunogenic anticancer activity cytokines of the present disclosure.

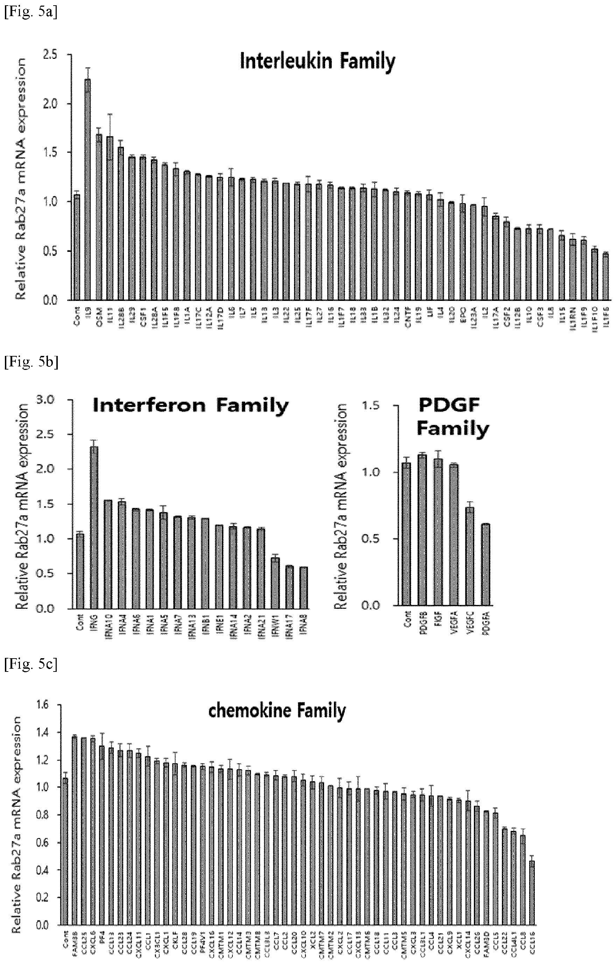

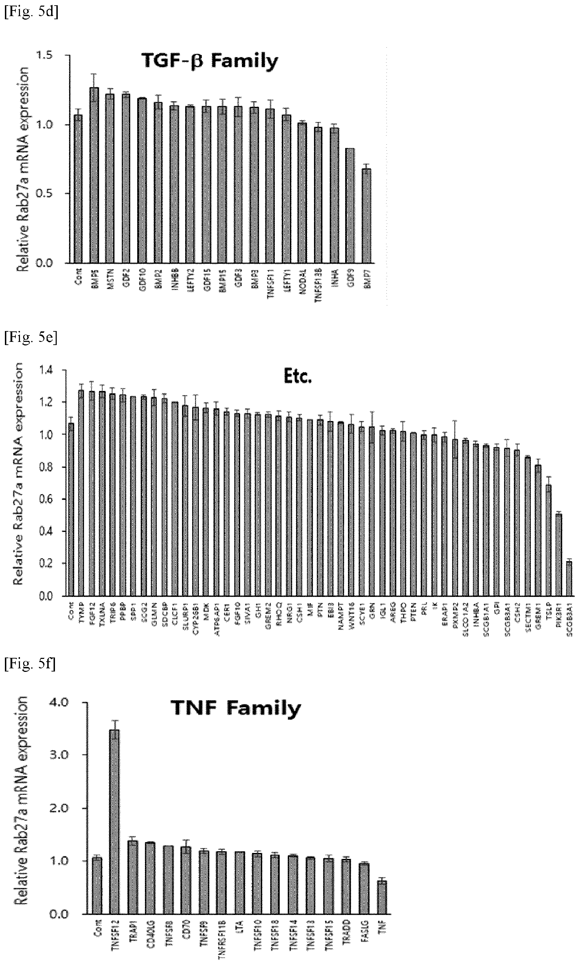

FIG. 5 shows results of classifying cancer cells with 202 kinds of cytokines expressed on the surface by family of cytokines according to a method for screening immunogenic anticancer activity cytokines of the present disclosure and identifying changes in the expression level of a gene associated with exosome secretion.
FIG. 6 shows results of isolating 202 kinds of cytokines according to a method for screening immunogenic anticancer activity cytokines of the present disclosure, and identifying cytokines thereamong that reduce expression of a gene associated with exosome secretion and commonly reduce expression of PD-L1.
FIG. 7 shows results of identifying that IL-15 selected by screening of the present disclosure reduces expression of PD-L1 in breast cancer cell lines.
FIG. 8 shows results of identifying that IL-15 selected by screening of the present disclosure reduces expression of exosome secretion-regulatory genes in breast cancer cell lines.
FIG. 9 shows results of identifying reduction in exosome expression by IL-15 in breast cancer cells.
FIG. 10 shows results of identifying that expression of exosomal PD-L1 was reduced by IL-15 in breast cancer cells.
FIG. 11 shows results of identifying apoptosis of breast cancer cells in co-culture of cytotoxic T cells and IL-15-treated breast cancer cells.
FIG. 12 shows results of identifying an increase in anticancer activity of cytotoxic T cells.
FIG. 13 shows results of identifying reduced expression of PD-1 and CTLA-4 by IL-15 in cytotoxic T cells.
FIG. 14 shows results of identifying changes in exosome secretion-associated genes and the exosome count in IL-15-treated cytotoxic T cells.
FIG. 15 shows results of identifying apoptosis of breast cancer cells in co-culture of IL-15-treated cytotoxic T cells and breast cancer cells.

### Best Mode for Carrying Out the Invention

The terms used herein have been selected from currently widely used general terms as much as possible in consideration of functions herein, but these may vary depending on the intentions or precedents of those skilled in the art, the emergence of new technologies, and the like. In addition, in specific cases, there are terms arbitrarily selected by the applicant, and in this case, the meaning will be described in detail in the description of the disclosure. Therefore, the terms used herein should not be defined by simple names of terms, but based on the meaning of the term and the overall contents of the present disclosure.

Unless otherwise defined, all terms used herein, including technical or scientific terms, have the same meaning as commonly understood by those skilled in the art to which the present disclosure pertains. Terms such as those defined in commonly used dictionaries should be construed as having meanings consistent with the meaning in the context of the relevant art and are not to be construed in an ideal or overly formal meaning unless clearly defined in the present application.

The numerical range includes the numerical value defined in the above range. All maximum numerical limits given herein include all lower numerical limits as clearly stated on the lower numerical limits. All minimum numerical limits given herein include all higher numerical limits as clearly stated on the higher numerical limits. All numerical limits given herein will include all better numerical ranges within a wider numerical range as clearly stated on narrower numerical limits.

Hereinafter, the present disclosure will be described in more detail.

The present disclosure provides a method for screening immunogenic anticancer activity cytokines, including (1) preparing a vector including a gene encoding a cytokine, a linker domain encoding a linker, and a transmembrane domain encoding a transmembrane protein; (2) preparing lentivirus including the vector prepared in operation (1); (3) transforming cancer cells with lentivirus prepared in operation (2); and, (4) when an expression level of a gene associated with exosome secretion or an expression level of PD-L1 in the cancer cells transformed in operation (3) decreases compared to untransformed control cells, determining that the cytokine in operation (1) has immunogenic anticancer activity.

The immunogenic anticancer activity is an activity of inhibiting exosome secretion or suppressing PD-L1 expression.

The cytokine may be any one or more selected from the group consisting of AREG, ATP6AP1, BMP10, BMP2, BMP3, BMP5, BMP7, BMP15, CCL1, CCL11, CCL13, CCL14, CCL16, CCL17, CCL18, CCL19, CCL2, CCL20, CCL21, CCL22, CCL23, CCL24, CCL25, CCL26, CCL28, CCL3, CCL3L1, CCL3L3, CCL4, CCL4L1, CCL5, CCL7, CCL8, CD40LG, CD70, CER1, CKLF, CLCF1, CMTM1, CMTM2, CMTM3, CMTM5, CMTM6, CMTM7, CMTM8, CNTF, CSF1, CSF2, CSF3, CSH1, CSH2, CX3CL1, CXCL1, CXCL10, CXCL11, CXCL12, CXCL13, CXCL14, CXCL16, CXCL2, CXCL3, CXCL6, CXCL9, CYP26B1, EBI3, EPO, ERAP1, FAM3B, FAM3D, FASLG, FGF10, FGF12, FIGF, GDF10, GDF15, GDF2, GDF3, GDF9, GH1, GLMN, GPI, GREM1, GREM2, GRN, IFNA1, IFNA10, IFNA13, IFNA14, IFNA17, IFNA2, IFNA21, IFNA4, IFNA5, IFNA6, IFNA7, IFNA8, IFNB1, IFNE1, IFNG, IFNW1, IGL1, IK, IL10, IL11, IL12A, IL12B, IL13, IL15, IL16, IL17A, IL17C, IL17D, IL17F, IL18, IL19, IL1A, IL1B, IL1F10, IL1F5, IL1F6, IL1F7, IL1F8, IL1F9, IL1RN, IL2, IL20, IL22, IL23A, IL24, IL25, IL27, IL28A, IL28B, IL29, IL3, IL32, IL33, IL4, IL5, IL6, IL7, IL8, IL9, INHA, INHBA, INHBB, LASS1, LEFTY1, LEFTY2, LIF, LTA, MDK, MIF, MSTN, NAMPT, NODAL, NRG1, OSM, PDGFA, PDGFB, PF4, PF4V1, PIK3R1, PPBP, PRL, PTEN, PTN, PXMP2, RHOQ, SCG2, SCGB1A1, SCGB3A1, SCYE1, SDCBP, SECTM1, SIVA1, SLCO1A2, SLURP1, SPP1, THPO, TNF, TNFRSF11B, TNFSF10, TNFSF12, TNFSF13, TNFSF14, TNFSF15, TNFSF18, TNFSF8, TNFSF9, TRADD, TRAP1, TRIP6, TSLP, TXLNA, TYMP, VEGFA, VEGFC, WNT16, XCL1, and XCL2.

The gene associated with the exosome secretion is Rab27a, and the expression level of Rab27a is measured using a primer set including a sequence represented by SEQ ID NO: 1 and a sequence represented by SEQ ID NO: 2.

The expression level of PD-L1 is measured using a primer set including a sequence represented by SEQ ID NO: 3 and a sequence represented by SEQ ID NO: 4.

The expression level of the various genes is measured using a primer set including sequences represented by SEQ ID NOS: 5 to 24.

The linker may have a 1-20 times repeating sequence of any one selected from among amino acid sequences represented by SEQ ID NOS: 25 to 35.

The transmembrane domain is a transmembrane domain included in any one receptor selected from the group consisting of an epidermal growth factor receptor, insulin receptor, platelet-derived growth factor (PDGF) receptor, vascular endothelial growth factor receptor, fibroblast growth factor receptor, cholecystokinin (CCK) receptor, neurotrophic factor (NGF) receptor, hepatocyte growth factor (HGF) receptor, ephrin (EPH) receptor, angiopoietin receptor, and related to receptor tyrosine kinase (RYK) receptor.

In addition, the present disclosure provides a pharmaceutical composition for preventing or treating cancer, including an immunogenic anticancer activity cytokine selected by the screening method as an active ingredient.

The cancer may be any one selected from the group consisting of melanoma, colon cancer, lung cancer, skin cancer, non-small cell lung cancer, bone cancer, pancreatic cancer, head or neck cancer, uterine cancer, ovarian cancer, rectal cancer, stomach cancer, anal cancer, breast cancer, fallopian tube carcinoma, endometrial carcinoma, cervical carcinoma, vaginal carcinoma, vulvar carcinoma, Hodgkin's disease, esophageal cancer, small intestine cancer, endocrine gland cancer, thyroid cancer, parathyroid cancer, adrenal cancer, soft tissue sarcoma, urethral cancer, penile cancer, prostate cancer, chronic or acute leukemia, lymphocytic lymphoma, bladder cancer, kidney or ureteral cancer, renal cell carcinoma, renal pelvic carcinoma, central nervous system tumor, primary central nervous system lymphoma, spinal cord tumor, brainstem glioma, and pituitary adenoma, but is not limited thereto.

The cytokine inhibits exosome secretion or suppresses expression of PD-L1 in cancer cells, wherein the PD-L1 is expressed in cancer cells or on the surface of exosomes generated from cancer cells.

The pharmaceutical composition may be formulated in the form of one or more external preparations selected from the group consisting of creams, gels, patches, sprays, ointments, emplastrum agents, lotions, liniments, pastas, and cataplasmas.

The pharmaceutical composition of the present disclosure may include a pharmaceutically acceptable carrier and diluent, which are additional for the formulation. The pharmaceutically acceptable carrier and diluent include excipients such as starch, sugar, and mannitol, fillers and extenders such as calcium phosphate, cellulose derivatives such as carboxymethylcellulose and hydroxypropyl cellulose, binders such as gelatin, alginate, and polyvinylpyrrolidone, lubricants such as talc, calcium stearate, hydrogenated castor oil, and polyethylene glycol, disintegrants such as povidone and crospovidone, and surfactants such as polysorbates, cetyl alcohol, and glycerol, but are not limited thereto. The pharmaceutically acceptable carrier and diluent may be biologically and physiologically compatible with subjects. Examples of the diluent may include saline, aqueous buffers, solvents, and/or dispersion media, but are not limited thereto.

The pharmaceutical composition of the present disclosure may be administered orally or parenterally (e.g., intravenously, subcutaneously, intraperitoneally, or topically) depending on a desired method, preferably orally. In addition, the dosage range of the pharmaceutical composition of the present disclosure varies depending on the subject's weight, age, sex, health condition, and diet, administration time, administration method, excretion rate, and severity of the disease, but is not limited thereto.

In addition, the present disclosure provides a health functional food composition including an immunogenic anticancer activity cytokine selected by the screening method as an active ingredient.

The present disclosure may be generally applied as a commonly used food product.

The food composition of the present disclosure may be used as a health functional food. The term "health functional food" as used herein refers to food manufactured and processed with raw materials or components having useful functionality for the human body in accordance with the Health Functional Food Act, and the term "functionality" as used herein refers to the intake for deriving useful effectiveness in health care such as regulation of nutrients or physiological actions for the structure and function of the human body.

The food composition of the present disclosure may include common food additives, and the suitability as the "food additive" is determined by the standards and criteria related to corresponding items according to the general rules and general test methods of Korean Food Additives Codex approved by the Ministry of Food and Drug Safety, unless otherwise stipulated.

The items listed in the "Korean Food Additives Codex" may include, for example, chemical compounds such as ketones, glycine, potassium citrate, nicotinic acid, and cinnamic acid, natural additives such as persimmon color, licorice extract, crystallized cellulose, kaoliang color, and guar gum, and mixed preparations such as sodium L-glutamate preparations, noodle-added alkali agents, preservative agents, and tar color agents.

The food composition of the present disclosure may be manufactured and processed in the form of tablets, capsules, powder, granules, liquids, and pills.

For example, hard capsule preparations among health functional foods in the form of capsule may be prepared by mixing and filling the composition according to the present disclosure in the conventional hard capsules along with additives such as excipients, and the soft capsule preparations may be manufactured by mixing the composition according to the present disclosure with the additives such as excipients and then filling the same in capsule bases such as gelatin. The soft capsule preparations may include, if necessary, plasticizers such as glycerin or sorbitol, colorants, and preservatives.

The definition of terms for the excipient, binder, disintegrant, lubricant, flavor enhancer, and flavoring agent is described in documents known in the art and includes those having the same or similar functions. The type of the food is not particularly limited and includes all health functional foods in the ordinary sense.

The term "prevention" as used herein refers to any action of suppressing or delaying diseases by administering the composition according to the present disclosure. The term "treatment" as used herein refers to any action that improves or favorably changes the symptoms of the disease by administering the composition according to the present disclosure. The term "improvement" as used herein refers to any action that improves the bad state of the disease by administering the composition of the present disclosure to an individual or making the individual intake the composition.

In addition, the present disclosure specifies that the expression or activity level of IL-15 may be measured by any one or more selected from the group consisting of reverse transcription-polymerase chain reaction (RT-PCR), western blotting, and fluorescence activated cell sorting (FACS), but is not limited thereto.

In addition, the present disclosure provides a method for inhibiting secretion of cancer cell-derived exosomes and PD-L1 expression, including administering IL-15, or an expression promoter or activator thereof to a subject.

In addition, the present disclosure provides a method for increasing secretion of immune cell-derived exosomes and inhibiting expression of PD-1 and CTLA-4, including administering IL-15, or an expression promoter or activator thereof to a subject.

### Modes for Carrying Out the Invention

Hereinafter, to help the understanding of the present disclosure, experimental examples and example embodiments will be described in detail. However, the following experimental examples and example embodiments are merely illustrative of the contents of the present disclosure, and the scope of the present disclosure is not limited to the following experimental examples and example embodiments. The experimental examples and example embodiments of the present disclosure are provided to more completely explain the present disclosure to those of ordinary skill in the art.

### Example 1: Cell Culture

HEK-293FT cells (human embryonic kidney) and MDA-MB-231 (human breast cancer cell line) were cultured in DMEM medium (Dulbecco's modified Eagle's medium, Hyclone) supplemented with 10% fetal bovine serum, 1% penicillin-streptomycin. 4T1 (mouse breast cancer cell line) was cultured in RPMI-1640 medium (Hyclone) supplemented with 10% FBS, 1% penicillin-streptomycin (Hyclone). CTLL-2 cells (mouse cytotoxic T lymphocytes) were cultured in RPMI 1640 medium supplemented with 10% FBS, 1% penicillin-streptomycin, and 20 IU/mL of recombinant IL-2 (R&D system).

### Example 2: Preparation of lentivirus

In order to screen immunogenic anticancer activity cytokines according to the present disclosure, a vector including a gene encoding 202 kinds of candidate cytokines predicted to exhibit immunogenic anticancer activity was prepared and transformed into cancer cells using lentivirus.

The vector including a gene encoding a cytokine, a linker domain encoding a linker, and a transmembrane domain encoding a transmembrane protein was prepared with reference to the paper (Proc Natl Acad Sci U S A. 2013 May 14;110(20):8099-104). Each vector was prepared to include each gene that encodes any one of 202 kinds of cytokines, including AREG, ATP6AP1, BMP10, BMP2, BMP3, BMP5, BMP7, BMP15, CCL1, CCL11, CCL13, CCL14, CCL16, CCL17, CCL18, CCL19, CCL2, CCL20, CCL21, CCL22, CCL23, CCL24, CCL25, CCL26, CCL28, CCL3, CCL3L1, CCL3L3, CCL4, CCL4L1, CCL5, CCL7, CCL8, CD40LG, CD70, CER1, CKLF, CLCF1, CMTM1, CMTM2, CMTM3, CMTM5, CMTM6, CMTM7, CMTM8, CNTF, CSF1, CSF2, CSF3, CSH1, CSH2, CX3CL1, CXCL1, CXCL10, CXCL11, CXCL12, CXCL13, CXCL14, CXCL16, CXCL2, CXCL3, CXCL6, CXCL9, CYP26B1, EBI3, EPO, ERAP1, FAM3B, FAM3D, FASLG, FGF10, FGF12, FIGF, GDF10, GDF15, GDF2, GDF3, GDF9, GH1, GLMN, GPI, GREM1, GREM2, GRN, IFNA1, IFNA10, IFNA13, IFNA14, IFNA17, IFNA2, IFNA21, IFNA4, IFNA5, IFNA6, IFNA7, IFNA8, IFNB1, IFNE1, IFNG, IFNW1, IGL1, IK, IL10, IL11, IL12A, IL12B, IL13, IL15, IL16, IL17A, IL17C, IL17D, IL17F, IL18, IL19, IL1A, IL1B, IL1F10, IL1F5, IL1F6, IL1F7, IL1F8, IL1F9, IL1RN, IL2, IL20, IL22, IL23A, IL24, IL25, IL27, IL28A, IL28B, IL29, IL3, IL32, IL33, IL4, IL5, IL6, IL7, IL8, IL9, INHA, INHBA, INHBB, LASS1, LEFTY1, LEFTY2, LIF, LTA, MDK, MIF, MSTN, NAMPT, NODAL, NRG1, OSM, PDGFA, PDGFB, PF4, PF4V1, PIK3R1, PPBP, PRL, PTEN, PTN, PXMP2, RHOQ, SCG2, SCGB1A1, SCGB3A1, SCYE1, SDCBP, SECTM1, SIVA1, SLCO1A2, SLURP1, SPP1, THPO, TNF, TNFRSF11B, TNFSF10, TNFSF12, TNFSF13, TNFSF14, TNFSF15, TNFSF18, TNFSF8, TNFSF9, TRADD, TRAP1, TRIP6, TSLP, TXLNA, TYMP, VEGFA, VEGFC, WNT16, XCL1, and XCL2.

The lentivirus to be used to transform cells with the vector was prepared in the following manner. HEK-293FT cells were inoculated, at a cell concentration of about 1 × 10⁶, in 6-well plates containing DMEM medium supplemented with 10% fetal bovine serum, 1% penicillin-streptomycin. In the presence of Opti-MEM (ref.31985-070; Gibco) medium, Lipofectamine 2000 Reagent (ref.11668-027; Thermo fisher scientific), pCMVD (# PS100001; Origene), and pVSVg (# 1733; Addgene) viral packaging vectors were treated by mixing with vectors including genes encoding cytokines in a ratio of 1:1:1, followed by overnight culture at 37°C. The next day, the supernatant was removed, and the medium was replaced with fresh medium including 10% fetal bovine serum, 1% penicillin-streptomycin. After 48 hours of culture, the virus-included supernatant of medium was obtained and centrifuged, and a surfactant-free cellulose acetate (SFCA) membrane filtration device (0.45 µm; Corning) was used to remove cell debris. The titer of lentivirus was measured by the Lenti-X p24 rapid titer kit (# 632200; Takara) according to the manufacturer's instructions. Lentiviruses were dispensed respectively and frozen at -80°C.

### Example 3: Transformation into cancer cells using lentivirus

In order to screen for cytokines exhibiting immunogenic anticancer activity according to the present disclosure, cancer cells were transformed with lentiviruses prepared in Example 2.

MDA-MB-231 cells, which are breast cancer cells, were inoculated in 48 well plates at a concentration of about 2.0 × 10⁴ cell/well. Lentivirus was treated with 10 µg/mL of polybrene at a concentration of 10 multiplicity of infection (MOI). The cell mixture treated with lentivirus was centrifuged at 1,200 g at room temperature for 90 minutes and then subjected to spinoculation, followed by overnight culture at 37°C. The surplus virus was removed, followed by medium replacement with fresh DMEM medium supplemented with 10% fetal bovine serum.

### Example 4: Changes in the expression level of Rab27a or PD-L1 in transformed cancer cells

Measured by real-time polymerase chain reaction (qRT-PCR) was whether the expression level of Rab27a, a gene associated with exosome secretion, or expression level of PD-L1 changed in transformed cancer cells according to the present disclosure.

After culturing the MDA-MB-231 cells transformed in Example 3 for 72 hours, mRNA was extracted from cells using an mRNA extraction kit (Direct-zol^{™} RNA Microprep, # R2060; Zymo Research) according to the manufacturer's instructions, followed by measurement of concentration of extracted mRNA using nanodrop (DS-11 Series Spectrophotometer; DeNovix). 500 ng of the extracted mRNA was synthesized into cDNA using a kit (Omniscript RT Kit, # 205113; QIAGEN) according to the manufacturer's instructions. To identify changes in the expression level of Rab27a or PD-L1 in each transformed cancer cell, qRT-PCR was performed for analysis using the primer set in Table 1 and Power SYBR^{™} Green PCR Master Mix (# 4367659; Applied Biosystems). StepOnePlus Real-Time PCR System (Applied Biosystems) instrument was used for qRT-PCR.

**TABLE 1**

| Gene | | Sequences | SEQ ID NO. |
|---|---|---|---|
| Huamn Rab27a | F | AAAAGGAGAAAGGGGCATGT | 1 |
| | R | TAATGGGGATGGTGAGAAGC | 2 |
| Human PD-L1 | F | GGTCATCCCAGAACTACCTC | 3 |
| | R | AGTGCTACACCAAGGCATAA | 4 |
| Human GAPDH | F | GAATTTGGCTACAGCAACAG | 5 |
| | R | TGAGGGTCTCTCTCITCCTC | 6 |
| Human Rab5 | F | GCATGGGTCCCTCTCACTAA | 7 |
| | R | GTGGAGAAATGGGCTGGTTA | 8 |
| Human Rab7 | F | CCCTGTTTGGATTGCAGAGT | 9 |
| | R | TGCAAATCAACGTTTTGGAA | 10 |
| Mouse Rab27a | F | TTCCTGCTTCTGTTCGACCT | 11 |
| | R | TTTCACTGCCCTCTGGTCTT | 12 |
| Mouse PD-L1 | F | AGTTCCCAAAACATGAGGAT | 13 |
| | R | CACGTACAAGTCCTTTGGAG | 14 |
| Mouse GAPDH | F | TCACCAC,CATGGAGAAGGC | 15 |
| | R | GCTAAGCAGTTGGTGGTGCA | 16 |
| Mouse Rab5 | F | TCTGCTGTTGGCAAATCAAG | 17 |
| | R | TCTCGCCAAAGGATTCCTCAT | 18 |
| Mouse Rab7 | F | GGCCTTCTACAGAGGTGCAG | 19 |
| | R | TCTTTGTGGCCACITGTCTG | 20 |
| Mouse PD-1 | F | AGAATCCTGGAGACCTCAAC | 21 |
| | R | ATACCCACTAGGGCACTCAT | 22 |
| Mouse CTLL-4 | F | GGACTCCGGAGGTACAAAGC | 23 |
| | R | TTGGGTCACCTGTATGGCTTC | 24 |

The expression level of Rab27a or PD-L1 in each sample was calculated by Ct (comparative threshold cycle) analysis after normalization to the amount of GAPDH in the same sample and was expressed as a modified 2^{-ΔΔCt} value from the initial Ct value.

### Example 5: Analysis on PD-L1 expression in IL-15-treated breast cancer cells

### 5-1. Real-time polymerase chain reaction

MDA-MB-231 cells, a human breast cancer cell line, were inoculated in 6-well plates at a concentration of about 4.0 × 10⁵ cell/well. 4T1 cells, a mouse breast cancer cell line, were inoculated at a concentration of 2.5 × 10⁵ cell/well, and IL-15 was treated at a concentration of 100 ng/ml the next day, followed by culture for 24 hours. After culture, mRNA was extracted from cells using mRNA extraction kit (Direct-zol^{™} RNA Microprep, # R2060; Zymo Research) according to the manufacturer's instructions, and the concentration of the extract mRNA was measured using nanodrop (DS-11 Series Spectrophotometer; DeNovix). 1000 ng of extracted mRNA was synthesized into cDNA using a kit (Omniscript RT Kit, # 205113; QIAGEN) according to the manufacturer's instructions. To identify the change in the expression level of PD-L1 in each transformed cancer cell, analysis was performed by qRT-PCR using the primer set shown in Table 1 and Power SYBR^{™} Green PCR Master Mix (# 4367659; Applied Biosystems). StepOnePlus Real-Time PCR System (Applied Biosystems) instrument was used for qRT-PCR.

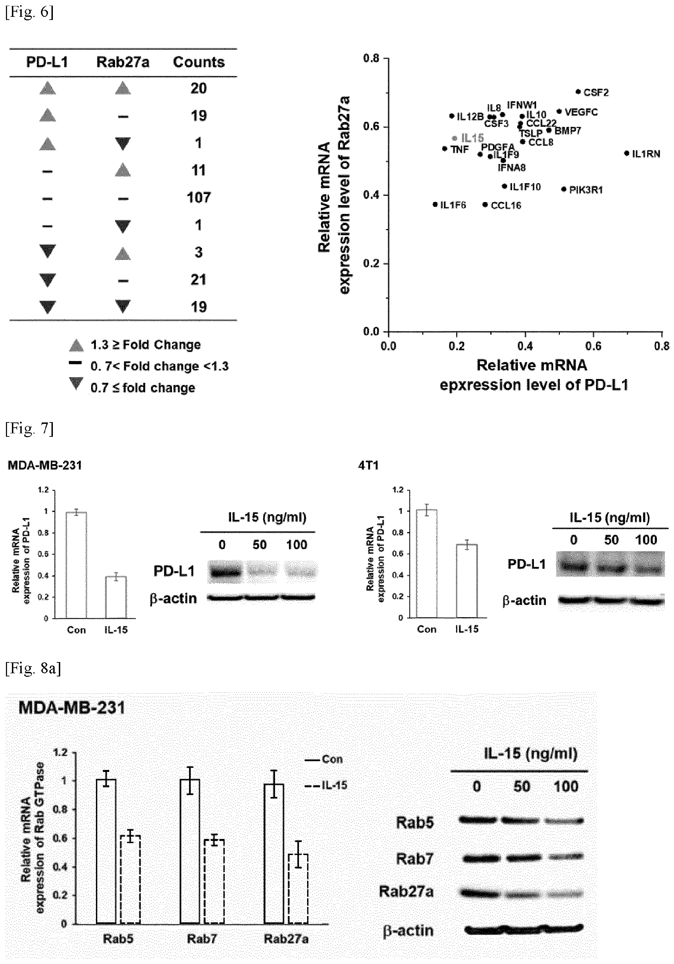

The expression level of PD-L1 in each sample was calculated by Ct (comparative threshold cycle) analysis after normalization to the amount of GAPDH in the same sample and was expressed as a modified 2^{-ΔΔCt} value from the initial Ct value. As a result, as shown in FIG. 7, it was found that PD-L1 mRNA expression significantly decreased in MDA-MB-231 and 4T1 cells treated with IL-15.

### 5-2. Western blot

The cells were lysed with a lysing buffer to prepare the cell lysate. The proteins of the cell lysate were then separated by SDS-PAGE and transferred to the nitrocellulose membrane. Thereafter, the membrane was blocked with 5% non-fat dry milk and 0.1% tween-20 blocking solution (TBS-T) at room temperature for 1 hour and subjected to a reaction with primary antibody of PD-L1 (ab210931; abcam) and beta-Actin (CST, #4970) and then with HRP-conjugated secondary antibody. Culture was performed overnight with respective primary antibody at 4°C and then with HRP-conjugated secondary antibody at room temperature for 1 hour. Enhanced chemiluminescence (ECL) detection reagent (# 34095; Thermo Scientific, # RPN2209; GE Healthcare) was used to visualize the image. As a result, as shown in FIG. 7, it was found that protein expression decreased in MDA-MB-231 and 4T1 cells treated with IL-15.

### Example 6: Analysis on exosome secretion-associated genes in IL-15-treated breast cancer cells

### 6-1. Real-time polymerase chain reaction and Western blot

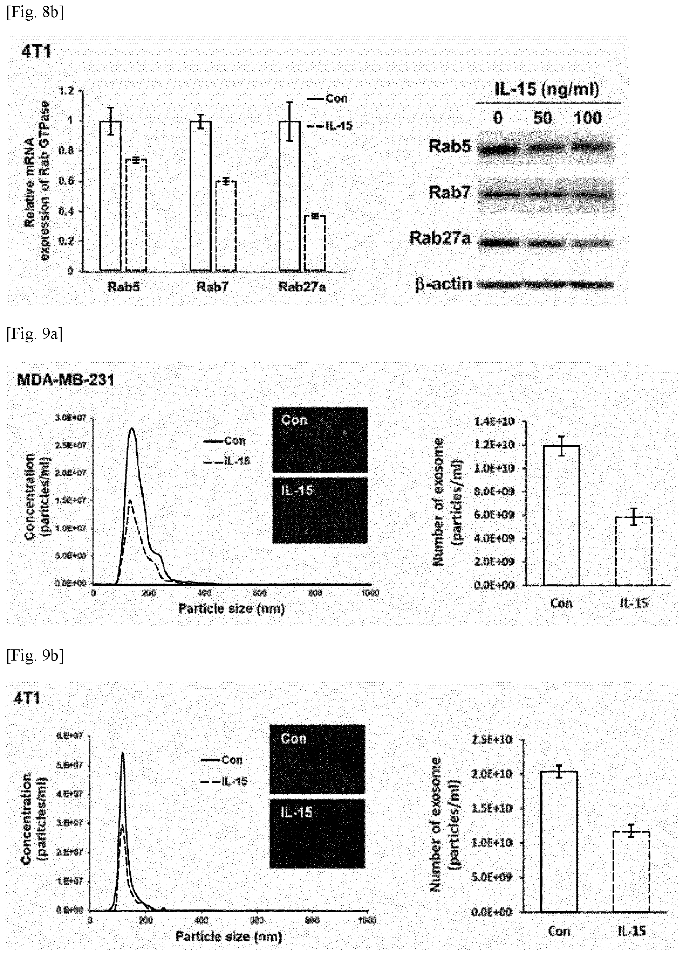

To analyze changes in exosomes secreted by MDA-MB-231 or 4T1 cells upon treatment of IL-15 (100 ng/ml), mRNA expression of enzymes (RabS, Rab7, Rab27a) involved in exosome production and secretion was measured. The real-time polymerase chain reaction was performed in the same manner as in Example 5-1, and primers shown in Table 1 were used. As a result, as shown in FIG. 8, it was found that mRNA expression of Rab5, Rab7, and Rab27a in IL-15-treated breast cancer cells decreased compared to the control group.

In addition, Western blot was performed using the cells. Western blot was performed in the same manner as in Example 5-2, and the primary antibodies used in this experiment are as follows: Rab5 (ab18211; abcam), Rab7 (ab50533; abcam), and Rab27a (ab55667; abcam). As a result, as shown in FIG. 8, it was found that protein expression of Rab5, Rab7, and Rab27a in IL-15-treated breast cancer cells decreased compared to the control group. From the above results, it was found that genes associated with the secretion of exosomes decreased in IL-15-treated breast cancer cells.

### Example 7: Isolation and purification of exosomes

Inoculation was performed on 150mm plates with about 6.4 × 10⁶ cells of MDA-MB-231 cells, a human breast cancer cell line, as well as 4.0 × 10⁶ cells of 4T1 cells, a mouse breast cancer cell line. The next day, inoculation was performed in fetal bovine serum-free DMEM and RPMI medium, and IL-15 was treated at a concentration of 100 ng/ml, followed by culture for 24 hours. The supernatant of the culture medium in which each cell was cultured was sequentially subjected to continuous centrifugation at 300 g, 2,500 g, and 10,000 g. The centrifuged supernatant was filtered through a 0.2 µm syringe filter and centrifuged at 120,000 g to obtain pellets of the extracellular vesicles. The obtained pellets were suspended in PBS and cryopreserved at -80°C.

### Example 8: Analysis on breast cancer cell-derived exosomes using nanoparticle tracking

The size and concentration of purified exosomes in breast cancer cells (MDA-MB-231, 4T1) were measured using Nanosight LM10 (Malvern Instruments) equipped with fast video capture and particle tracking software. As a result, as shown in FIG. 9, it was determined by nanoparticle tracking analysis that the number of exosomes decreased in two types of breast cancer cells treated with IL-15 compared to the control group.

### Example 9: Analysis on expression regulation for exosomal PD-L1 derived from IL-15-treated breast cancer cells

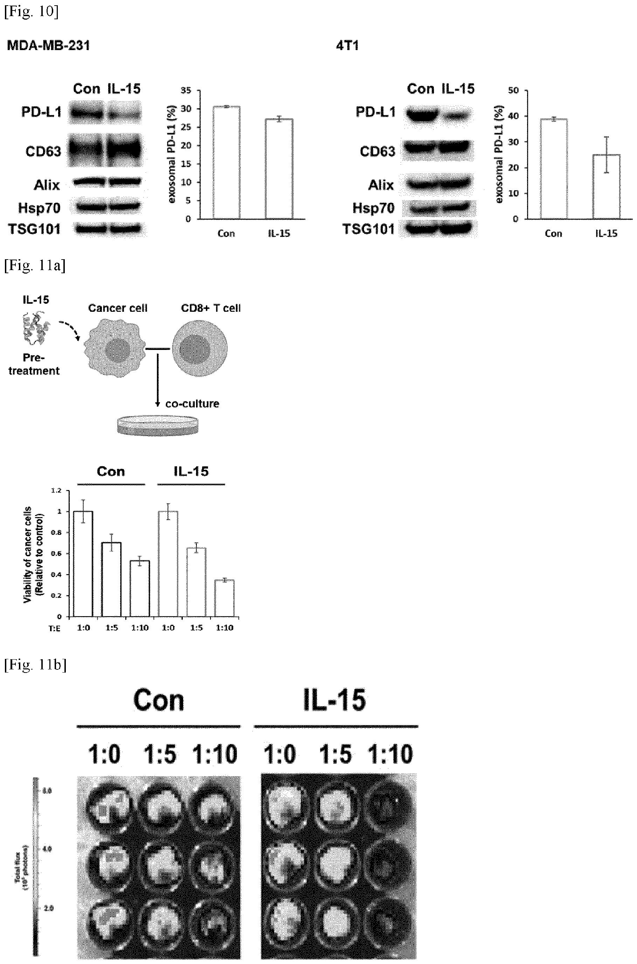

To evaluate the efficacy of IL-15 in breast cancer cells (MDA-MB-231, 4T1), measured was expression of PD-L1 protein in exosomes, which is known to be the most important for immune evasion of cancer cells. Western blot was performed using the exosome. Western blot was performed in the same manner as in Example 5-2. As a result, as shown in FIG. 10, it was found that protein expression of PD-L1 significantly decreased in two types of breast cancer cells treated with IL-15 compared to the control group.

The exosome was cultured overnight with an anti-PD-L1 (ab10931; abcam) antibody and then with FITC-conjugated secondary antibody (a11001; invitrogen/goat antimouse igg (h+1) cross-adsorbed secondary antibody alexa fluor 488), followed by analysis using sEV flow cytometer (CytoFlEX/Beckman Coulter). As a result, as shown in FIG. 10, it was found that the expression amount of exosomal PD-L1 in the IL-15-treated sample decreased compared to the control group.

### Example 10: Analysis on co-culture of IL-15-pretreated breast cancer cells

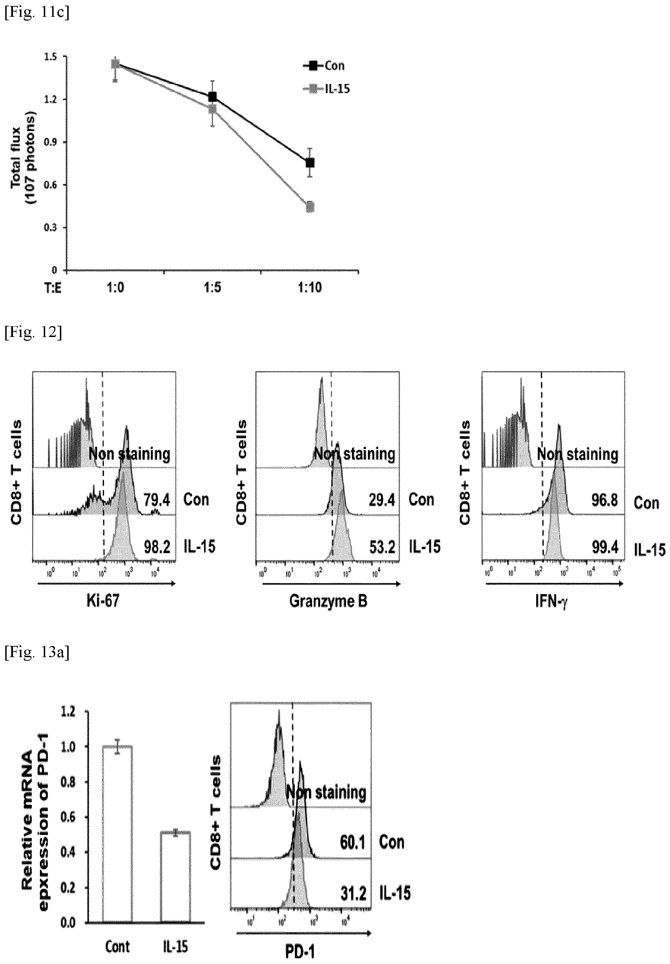

In order to prove the anticancer effect of IL-15 by a decrease of PD-L1 in breast cancer cells as well as an increase in the activity of cytotoxic T cells, IL-15-treated breast cancer cells 4T1 and cytotoxic T cells were co-cultured to identify the viability of breast cancer cells as shown in FIG. 11. Breast cancer cells (4T1-luc-g5) having cytoluminescent genes were used, and analysis was performed using MTS and IVIS spectrum (PerkinElmer).

Breast cancer cells were inoculated in 96-well plates at a density of 6.0 × 10³ cell/well and pretreated with IL-15 (100 ng/ml). After 24 hours of culture, the cells were cultured with different proportions of CTLL-2 cells (1:5 to 1:10). At the end of the culture, MTS reagent was added to each well, followed by culture at 37°C for 4 hours. A microplate reader was used to measure absorbance at 490 nm.

In addition, to analyze the viability of 4T1-luc-g5 cells, a luminescent and fluorescence animal in vivo imaging system (IVIS) was used. D-luciferin (PIN 122799; Perkin) was added to each well, and luciferin was measured using IVIS software. As a result, as shown in FIG. 11, it was found that the viability of breast cancer cells significantly decreased in the co-culture group of cytotoxic T cells and IL-15-treated breast cancer cells.

### Example: 11 Analysis on the anticancer efficacy in IL-15-activated CTLL-2 cells

Next, cytotoxic T cells were treated with IL-15 to evaluate the efficacy of exosomes. Briefly, CTLL-2 cells were inoculated in 6-well plates at a density of 2.0 × 10⁵ cell/well, followed by culture with IL-15 (100 ng/ml) for 24 hours. Thereafter, flow cytometry was performed on Ki-67, IFN-γ, perforin, and granzyme B. The primary antibodies used in this experiment are as follows: Ki-67 (ab16667, 1:500; Abcam), granzyme B (ab4059, 1:500 Abcam), and IFN-g (ab9657, 1:500; Abcam). As a result, as shown in FIG. 12, when CTLL-2 cells were treated with IL-15, it was found that expression of Ki-67 protein, an indicator of cell proliferation, increased compared to the control group.

In addition, when CTLL-2 cells were treated with IL-15, it was found that expression of IFN-γ and granzyme B proteins which are indicators of cytotoxicity also increased compared to the control group. Therefore, it was determined that IL-15 enhanced the cytotoxic function of cytotoxic T cells, thereby improving the anticancer effect.

### Example 12: Analysis on the immune checkpoint in IL-15-activated CTLL-2 cell s

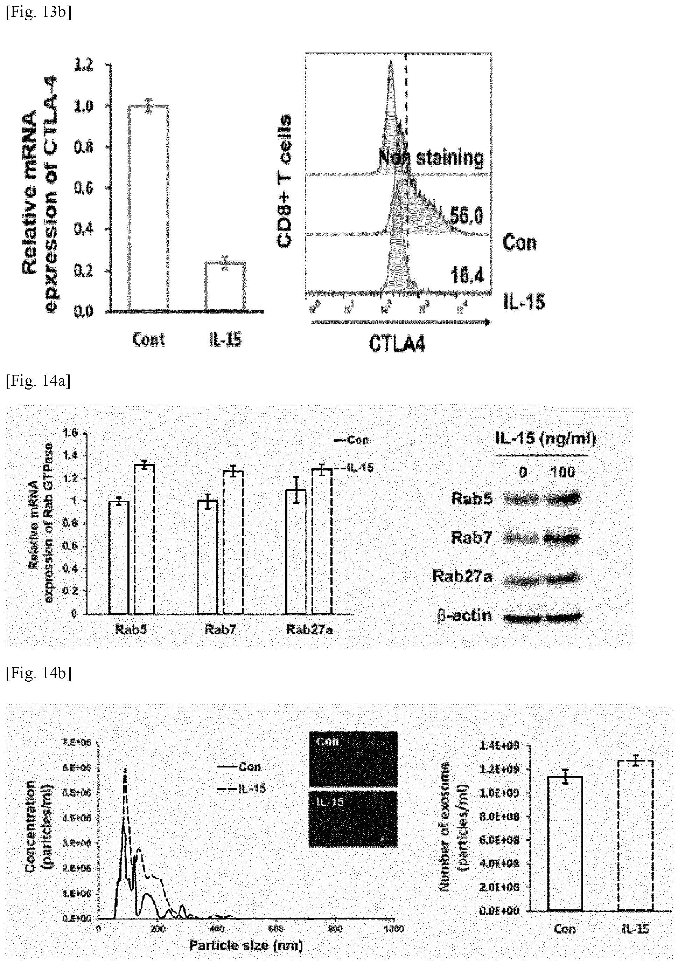

Next, cytotoxic T cells were treated with IL-15 to evaluate changes in expression of PD-1 and CTLA-4, which are immune checkpoints. Moreover, expression of the immune checkpoint PD-1 (programmed cell death-1) and CTLA-4 (cytotoxic T lymphocyte antigen-4) in IL-15-treated cytotoxic T cells was identified in the same manner as in Examples 5-1 and 11. As a result, as shown in FIG. 13, it was found that mRNA and protein expression of PD-1 and CTLA-4 significantly decreased in cytotoxic T cells treated with IL-15 compared to the control group. This suggests that the decreased PD-1 and CTLA-4 in cytotoxic T cells caused reduction of immune evasion due to the binding of cancer cells to PD-L1, thereby enhancing the anticancer effect.

### Example 13: Analysis on changes in exosome secretion in IL-15-treated cytotoxic T cells

The expression of exosome secretion-associated genes Rab5, Rab7, and Rab27a in IL-15-treated CTLL2 cells was analyzed by qRT-PCR and Western blot. As a result, as shown in FIG. 14, it was found that mRNA and protein expression of Rab5, Rab7 and Rab27a significantly increased in IL-15-treated cytotoxic T cells compared to the control group.

In addition, analyzed by NTA was that the exosomes secreted from IL-15-treated CTLL-2 cells increased compared to the control group. As a result, as shown in FIG. 14, it was found that exosome expression increased in cytotoxic T cells treated with IL-15 compared to the control group. This suggests that the anticancer effect may be enhanced by increasing exosomes of cytotoxic T cells.

### Example 14: Analysis on co-culture of IL-15-pretreated T cells

Breast cancer cells were inoculated in 96-well plates at a density of about 6.0 × 10³ cell/well, and CTLL-2 cells pretreated with IL-15 for 24 hours were co-cultured with different proportions of melanoma cells (1:5 to 1:10). At the end of culture, MTS reagent was added to each well and cultured at 37°C for 4 hours. A microplate reader was used to measure absorbance at 490 nm.

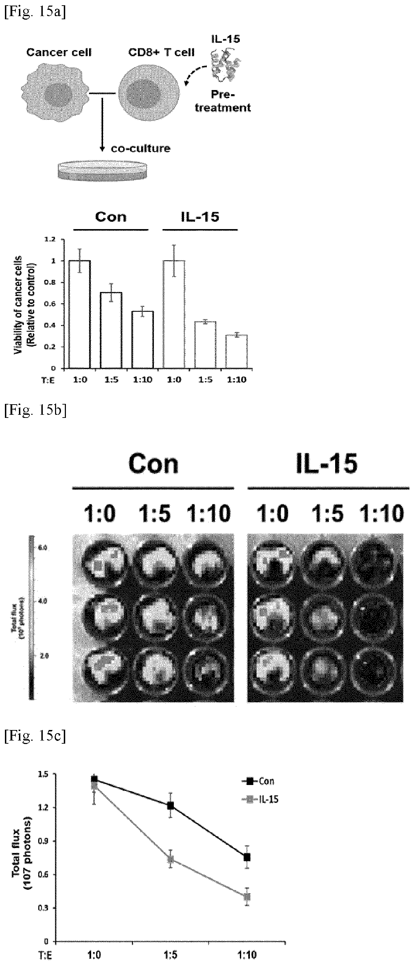

In addition, to analyze the viability of 4T1-luc-g5 cells, a luminescent and fluorescence animal in vivo imaging system (IVIS) was used. D-luciferin (PIN 122799; Perkin) was added to each well, and luciferin was measured using IVIS software. As a result, as shown in FIG. 15, it was found that the viability of breast cancer cells was significantly reduced in the co-culture group of IL-15-pretreated cytotoxic T cells and breast cancer cells.

As described above, a specific part of the content of the present disclosure is described in detail, for those of ordinary skill in the art, it is clear that the specific description is only a preferred embodiment, and the scope of the present disclosure is not limited thereby. In other words, the substantial scope of the present disclosure may be defined by the appended claims and their equivalents.

## Claims

1. A method for screening immunogenic anticancer activity cytokines, comprising:
(1) preparing a vector comprising a gene encoding a cytokine, a linker domain encoding a linker, and a transmembrane domain encoding a transmembrane protein;
(2) preparing lentivirus comprising the vector prepared in operation (1);
(3) transforming cancer cells with lentivirus prepared in operation (2); and,
(4) when an expression level of a gene associated with exosome secretion or an expression level of PD-L1 in the cancer cells transformed in operation (3) decreases compared to untransformed control cells, determining that the cytokine in operation (1) has immunogenic anticancer activity.

2. The method of claim 1, wherein the immunogenic anticancer activity is an activity of inhibiting exosome secretion or suppressing PD-L1 expression.

3. The method of claim 1, wherein the cytokine is any one or more selected from the group consisting of AREG, ATP6AP1, BMP10, BMP2, BMP3, BMP5, BMP7, BMP15, CCL1, CCL11, CCL13, CCL14, CCL16, CCL17, CCL18, CCL19, CCL2, CCL20, CCL21, CCL22, CCL23, CCL24, CCL25, CCL26, CCL28, CCL3, CCL3L1, CCL3L3, CCL4, CCL4L1, CCL5, CCL7, CCL8, CD40LG, CD70, CER1, CKLF, CLCF1, CMTM1, CMTM2, CMTM3, CMTM5, CMTM6, CMTM7, CMTM8, CNTF, CSF1, CSF2, CSF3, CSH1, CSH2, CX3CL1, CXCL1, CXCL10, CXCL11, CXCL12, CXCL13, CXCL14, CXCL16, CXCL2, CXCL3, CXCL6, CXCL9, CYP26B1, EBI3, EPO, ERAP1, FAM3B, FAM3D, FASLG, FGF10, FGF12, FIGF, GDF10, GDF15, GDF2, GDF3, GDF9, GH1, GLMN, GPI, GREM1, GREM2, GRN, IFNA1, IFNA10, IFNA13, IFNA14, IFNA17, IFNA2, IFNA21, IFNA4, IFNA5, IFNA6, IFNA7, IFNA8, IFNB1, IFNE1, IFNG, IFNW1, IGL1, IK, IL10, IL11, IL12A, IL12B, IL13, IL15, IL16, IL17A, IL17C, IL17D, IL17F, IL18, IL19, IL1A, IL1B, IL1F10, IL1F5, IL1F6, IL1F7, IL1F8, IL1F9, IL1RN, IL2, IL20, IL22, IL23A, IL24, IL25, IL27, IL28A, IL28B, IL29, IL3, IL32, IL33, IL4, IL5, IL6, IL7, IL8, IL9, INHA, INHBA, INHBB, LASS1, LEFTY1, LEFTY2, LIF, LTA, MDK, MIF, MSTN, NAMPT, NODAL, NRG1, OSM, PDGFA, PDGFB, PF4, PF4V1, PIK3R1, PPBP, PRL, PTEN, PTN, PXMP2, RHOQ, SCG2, SCGB1A1, SCGB3A1, SCYE1, SDCBP, SECTM1, SIVA1, SLCO1A2, SLURP1, SPP1, THPO, TNF, TNFRSF11B, TNFSF10, TNFSF12, TNFSF13, TNFSF14, TNFSF15, TNFSF18, TNFSF8, TNFSF9, TRADD, TRAP1, TRIP6, TSLP, TXLNA, TYMP, VEGFA, VEGFC, WNT16, XCL1, and XCL2.

4. The method of claim 1, wherein the gene associated with exosome secretion is Rab27a.

5. The method of claim 4, wherein the expression level of Rab27a is measured using a primer set comprising a sequence represented by SEQ ID NO: 1 and a sequence represented by SEQ ID NO: 2.

6. The method of claim 1, wherein the expression level of the PD-L1 is measured using a primer set comprising a sequence represented by SEQ ID NO: 3 and a sequence represented by SEQ ID NO: 4.

7. The method of claim 1, wherein the linker has a 1-20 times repeating sequence of any one selected from among amino acid sequences represented by SEQ ID NOS: 25 to 35.

8. The method of claim 1, wherein the transmembrane domain is a transmembrane domain included in any one receptor selected from the group consisting of an epidermal growth factor receptor, insulin receptor, platelet-derived growth factor (PDGF) receptor, vascular endothelial growth factor receptor, fibroblast growth factor receptor, cholecystokinin (CCK) receptor, neurotrophic factor (NGF) receptor, hepatocyte growth factor (HGF) receptor, ephrin (EPH) receptor, angiopoietin receptor, and related to receptor tyrosine kinase (RYK) receptor.

9. A pharmaceutical composition for preventing or treating cancer, comprising an immunogenic anticancer activity cytokine selected by the screening method of any one of claims 1 to 8 as an active ingredient.

10. The pharmaceutical composition of claim 9, wherein the cancer is any one selected from the group consisting of melanoma, colon cancer, lung cancer, skin cancer, non-small cell lung cancer, bone cancer, pancreatic cancer, head or neck cancer, uterine cancer, ovarian cancer, rectal cancer, stomach cancer, anal cancer, breast cancer, fallopian tube carcinoma, endometrial carcinoma, cervical carcinoma, vaginal carcinoma, vulvar carcinoma, Hodgkin's disease, esophageal cancer, small intestine cancer, endocrine gland cancer, thyroid cancer, parathyroid cancer, adrenal cancer, soft tissue sarcoma, urethral cancer, penile cancer, prostate cancer, chronic or acute leukemia, lymphocytic lymphoma, bladder cancer, kidney or ureteral cancer, renal cell carcinoma, renal pelvic carcinoma, central nervous system tumor, primary central nervous system lymphoma, spinal cord tumor, brainstem glioma, and pituitary adenoma.

11. The pharmaceutical composition of claim 9, wherein the cytokine is any one or more selected from the group consisting of AREG, ATP6AP1, BMP10, BMP2, BMP3, BMP5, BMP7, BMP15, CCL1, CCL11, CCL13, CCL14, CCL16, CCL17, CCL18, CCL19, CCL2, CCL20, CCL21, CCL22, CCL23, CCL24, CCL25, CCL26, CCL28, CCL3, CCL3L1, CCL3L3, CCL4, CCL4L1, CCL5, CCL7, CCL8, CD40LG, CD70, CER1, CKLF, CLCF1, CMTM1, CMTM2, CMTM3, CMTM5, CMTM6, CMTM7, CMTM8, CNTF, CSF1, CSF2, CSF3, CSH1, CSH2, CX3CL1, CXCL1, CXCL10, CXCL11, CXCL12, CXCL13, CXCL14, CXCL16, CXCL2, CXCL3, CXCL6, CXCL9, CYP26B1, EBI3, EPO, ERAP1, FAM3B, FAM3D, FASLG, FGF10, FGF12, FIGF, GDF10, GDF15, GDF2, GDF3, GDF9, GH1, GLMN, GPI, GREM1, GREM2, GRN, IFNA1, IFNA10, IFNA13, IFNA14, IFNA17, IFNA2, IFNA21, IFNA4, IFNA5, IFNA6, IFNA7, IFNA8, IFNB1, IFNE1, IFNG, IFNW1, IGL1, IK, IL10, II,11, IL12A, IL12B, IL13, IL15, IL16, IL17A, IL17C, IL17D, IL17F, IL18, IL19, II,1A, IL1B, IL1F10, IL1F5, IL1F6, IL1F7, IL1F8, IL1F9, IL1RN, IL2, IL20, IL22, IL23A, IL24, IL25, IL27, IL28A, IL28B, IL29, IL3, IL32, IL33, IL4, IL5, IL6, IL7, IL8, IL9, INHA, INHBA, INHBB, LASS1, LEFTY1, LEFTY2, LIF, LTA, MDK, MIF, MSTN, NAMPT, NODAL, NRG1, OSM, PDGFA, PDGFB, PF4, PF4V1, PIK3R1, PPBP, PRL, PTEN, PTN, PXMP2, RHOQ, SCG2, SCGB1A1, SCGB3A1, SCYE1, SDCBP, SECTM1, SIVA1, SLCO1A2, SLURP1, SPP1, THPO, TNF, TNFRSF11B, TNFSF10, TNFSF12, TNFSF13, TNFSF14, TNFSF15, TNFSF18, TNFSF8, TNFSF9, TRADD, TRAP1, TRIP6, TSLP, TXLNA, TYMP, VEGFA, VEGFC, WNT16, XCL1, and XCL2.

12. The pharmaceutical composition of claim 9, wherein the cytokine inhibits exosome secretion or suppresses expression of PD-L1 in cancer cells.

13. The pharmaceutical composition of claim 12, wherein the PD-L1 is expressed in cancer cells or on surfaces of exosomes generated from cancer cells.

14. A health functional food composition, comprising an immunogenic anticancer activity cytokine selected by the screening method of any one of claims 1 to 8 as an active ingredient.

15. A pharmaceutical composition for preventing or treating a cancer disease, comprising IL-15, or an expression promoter or activator thereof as an active ingredient.

16. The pharmaceutical composition of claim 15, wherein the IL-15 expression promoter or activator is selected from the group consisting of compounds, peptides, aptamers, and antibodies that specifically bind to IL-15 proteins.

17. The pharmaceutical composition of claim 15, wherein the IL-15, or the expression promoter or activator thereof reduces cancer cell-derived exosome secretion and expression of PD-L1 to inhibit immune evasion and enhance an anticancer effect.

18. The pharmaceutical composition of claim 15, wherein the IL-15, or the expression promoter or activator thereof enhances an activity of cytotoxic T cells, reduces PD-1 and CTLA-4, which are immune checkpoints, inhibits immune evasion through promotion of exosome secretion, and enhances an anticancer effect.

19. The pharmaceutical composition of claim 15, wherein the cancer disease is selected from the group consisting of melanoma, skin cancer, lung cancer, liver cancer, gastric cancer, pancreatic cancer, bone cancer, head or neck cancer, uterine cancer, ovarian cancer, breast cancer, fallopian tube carcinoma, endometrial carcinoma, cervical carcinoma, vaginal carcinoma, vulvar carcinoma, Hodgkin's disease, esophageal cancer, small intestine cancer, colorectal cancer, colon cancer, rectal cancer, anal cancer, endocrine gland cancer, thyroid cancer, parathyroid cancer, adrenal cancer, soft tissue sarcoma, urethral cancer, penile cancer, prostate cancer, chronic or acute leukemia, lymphocytic lymphoma, bladder cancer, kidney or ureteral cancer, renal cell carcinoma, renal pelvic carcinoma, central nervous system tumors, primary central nervous system lymphoma, spinal cord tumor, brainstem glioma, and pituitary adenoma.
